# EUROPEAN PATENT SPECIFICATION

(11) **EP 1 668 003 B1**
(45) Date of publication and mention of the grant of the patent: **14.11.2007**
(21) Application number: 04787069.6
(22) Date of filing: 30.09.2004
(51) Int. Cl.: C07D 413/04, A61K 31/454, A61P 25/18

(54) **Benzisoxazoles**
Benzisoxazole
Benzisoxazoles

(30) Priority: 01.10.2003 GB 0322994
(43) Date of publication of application: 14.06.2006
(73) Proprietor: Novartis AG, 4056 Basel (CH); Novartis Pharma GmbH, 1230 Wien (AT)
(72) Inventor: NOZULAK, Joachim, 79423 Heitersheim (DE); KALKMAN, Hans, O., CH-4053 Basel (CH)
(74) Representative: Hofstetter, Alfons J.
(86) International application number: PCT/EP2004/010938
(87) International publication number: WO 2005/030763

(56) References cited:
- EP-A- 0 402 644
- WO-A-98/07938
- US-A- 5 955 459
- US-B1- 6 258 836
- STRUPCZEWSKI J T ET AL: "3-(ARYLOXY)ALKYLPIPERIDINYL-1,2-BENZISOXA ZOLES AS D2/5-HT2 ANTAGONISTS WITH POTENTIAL ATYPICAL ANTIPSYCHOTIC ACTIVITY: ANTIPSYCHOTIC PROFILE OF ILOPERIDONE (HP 873)" JOURNAL OF MEDICINAL CHEMISTRY, AMERICAN CHEMICAL SOCIETY. WASHINGTON, US, vol. 38, no. 7, 1995, pages 1119-1131, XP000941571 ISSN: 0022-2623
- KELLEHER J P ET AL: "ADVANCES IN ATYPICAL ANTIPSYCHOTICS FOR THE TREATMENT OF SCHIZOPHRENIA NEW FORMULATIONS AND NEW AGENTS" CNS DRUGS, ADIS INTERNATIONAL, AUCKLAND, NZ, vol. 16, no. 4, 2000, pages 249-261, XP001079584 ISSN: 1172-7047

## Description

The present invention relates to novel fatty acid esters of the reversible Iloperidone metabolite P-88-8991, their preparation, their use as pharmaceuticals and pharmaceutical compositions containing them.

Ilioperidone and a genus of compounds comprising iloperidone are taught in EP-A-0 402 644. Iloperidone is an atypical antipsychotic developed for the treatment of schizophrenia, having relevant affinity for noradrenergic, dopaminergic and serotoninergic receptors. See for example Richelson E. and Souder T., Life Sciences, 68:29-39 (2000). See, also, Strupczewski et al., J. Med. Chem., 38 (7):1119-1131 (1995) and Kelleher et al., CNS Drugs, ADIS International, 16(4): 249-261 (2000). US-B1-6 258 836 discloses conjugation of fatty acids to drugs. US-B1-5 955 459 to Bradley et al. discloses fatty acid amide derivatives of clozapine, an atypical antipsychotic, as well as the derivation of other antipsychotics, including iloperidone. Iloperidone is metabolized in a reversible manner to P-88-8991 having the formula II

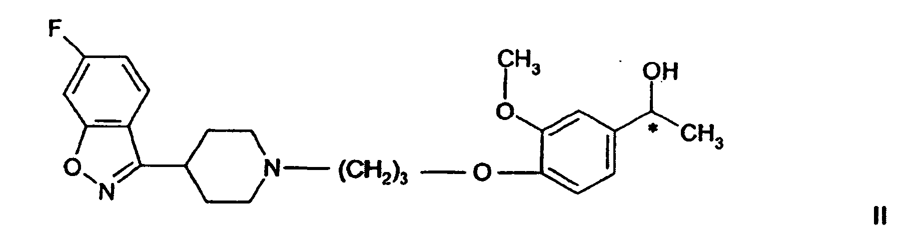

See for example Mutlib AE et al., Drug Metab. Dispos; 23 (9): 951-964 (1995). P-88-8991 has been shown to have plasma levels in human about 1.5 fold higher than the parent drug. It is roughly as active as Iloperidone.

More particularly, the invention relates to compounds of formula I 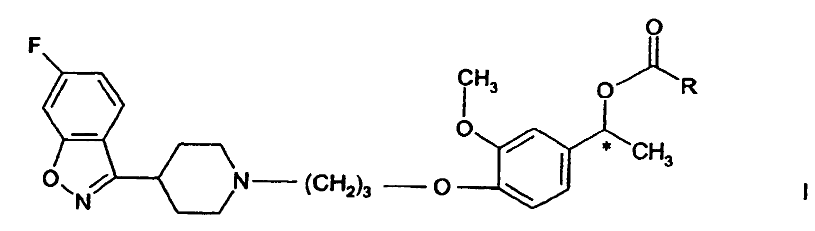 wherein R is (C₁₋₄₀)alkyl or (C₁₋₄₀)alkenyl, in free base or acid addition salt form.

On account of the asymmetrical carbon atom which is present in the compounds of formula I, the compounds may exist in optically active form or in form of mixtures of optical isomers,
e.g. in form of racemic mixtures. All optical isomers and their mixtures including the racemic mixtures are part of the present invention.

In a further aspect, the invention provides a process for the production of the compounds of formula I and their salts, comprising the step of reacting the metabolite P-88-8991 of formula 11 with a compound of formula III 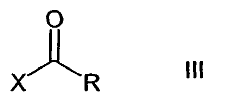 wherein R is as defined above and X is halogen, and recovering the so obtained compound of formula I in free base or acid addition salt form.

The reaction can be effected according to conventional methods, e.g. as described in Example 1.

In formula III, X is preferably chlorine or bromine.

Working up the reaction mixtures and purification of the compounds thus obtained may be carried out in accordance to known procedures.

Acid addition salts may be produced from the free bases in known manner, and vice-versa. Suitable acid addition salts for use in accordance with the present invention include for example the hydrochloride.

The starting compounds of formula II may be obtained by reducing Iloperidone of formula IV 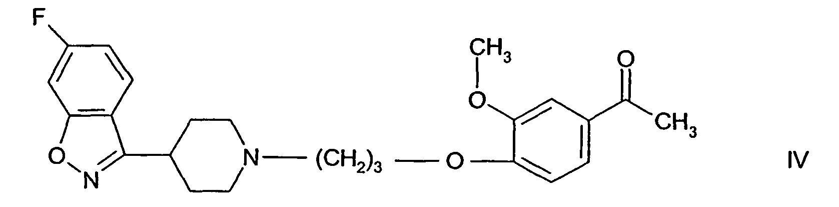 with an enantiomer of the boran complex of formula V

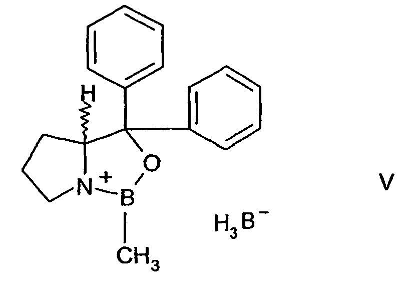

The compound of formula II (S)-1-(4-{3-[4-(6-fluoro-benzo[d]isoxazol-3-yl)-piperidin-1-yl]propoxy}-3-methoxy-penyl)-ethanol is obtained using the reagent (R)-2-methyl-CBS-oxazaborolidine-borane complex of formula Va 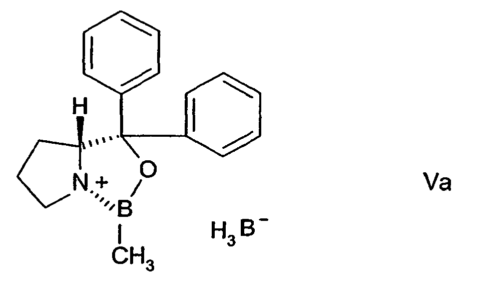 whereas the compound of formula II (R)-1-(4-{3-[4-(6-fluoro-benzo[d]isoxazol-3-yl)-piperidin-1-yl]propoxy}-3-methoxy-phenyl)-ethanol is obtained using the reagent (S)-2-methyl-CBS-oxazaborolidine-borane complex of formula Vb

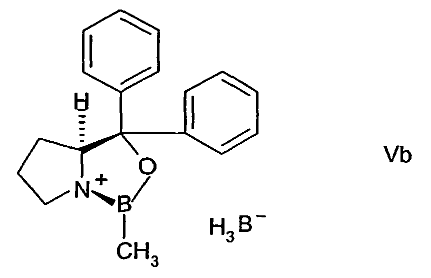

The reactions can be effected according to conventional methods, e.g. as described in Example 1.

The boran complexes used as starting materials can be produced from the corresponding compounds of formula Vla and Vlb 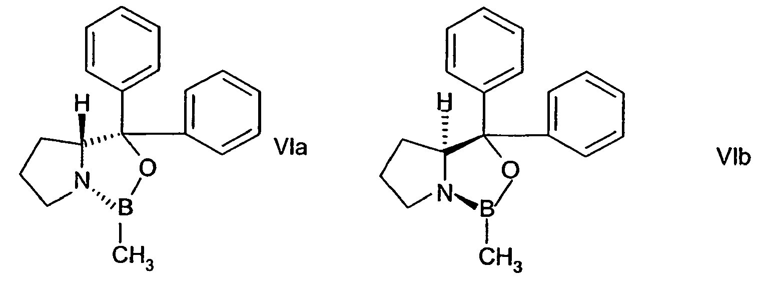 according to known procedures, e.g. as described in Example 1.

The starting materials of formulae VIa and VIb are known (for a review of these catalysts, see Corey, E. et al., Angew. Chem., Int. Ed. Engl. 1998, 37, 1986).

The compounds of formula I and their pharmaceutically acceptable acid addition salts, hereinafter referred to as agents of the invention, exhibit valuable pharmacological properties when tested in animals, and are therefore useful as pharmaceuticals.

In particular, the agents of the invention exhibit antipsychotic and anti-manic activity, as assessed in standard tests such as the amphetamine-induced hypermotility and the phencyclidine-induced hyperlocomotion tests.

The amphetamine-induced hypermotility test is performed according to the method described by Arnt J in Eur. J. Pharmacol. 283, 55-62 (1995). In this test, the agents of the invention significantly inhibit the amphetamine-induced locomotion of the animals at doses of about 0.01 to about 10 mg/kg s.c.

The phencyclidine-induced hyperlocomotion test is performed according to a rat adaptation of the method described by Gleason SD and Shannon HE in Psychopharmacol. 129, 79-84 . (1997). In this test, the agents of the invention significantly block the phencyclidine-induced hyperlocomotion of the rats at doses of about 0.01 to about 10 mg/kg s.c.

The agents of the invention are therefore useful for the treatment of psychotic disorders such as schizophrenia and bipolar disorders.

It has been found that the agents of the invention are enzymatically metabolized into the active compound of formula II which is believed to be predominantly responsible of the in vivo activity in the above-mentioned tests. This ester cleavage has been found to proceed at slow rate. The agents of the invention are therefore of particular interest for use in pharmaceutical compositions aimed at providing a slow release of the compound of formula II.

For the above-mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 500, preferably from about 0.5 to about 100 mg/kg animal body weight. In larger mammals, for example humans, an indicated daily dosage is in the range from about 10 to about 2000, preferably from about 100 to about 1000 mg of an agent of the invention, conveniently administered, for example, in divided doses up to four times a day or in sustained release form.

The agent of the invention may be administered by any conventional route, preferably parenterally, for example in the form of injectable solutions or suspensions for intramuscular administration, or transdermally.

In accordance with the foregoing, the present invention also provides an agent of the invention, for use as a pharmaceutical, e.g. for the treatment of psychotic disorders.

The present invention furthermore provides a pharmaceutical composition comprising an agent of the invention in association with at least one pharmaceutical carrier or diluent. Such compositions may be manufactured in conventional manner. Unit dosage forms contain, for example, from about 0.25 to about 150, preferably from 0.25 to about 25 mg of a compound according to the invention.

Moreover the present invention provides the use of an agent of the invention, for the manufacture of a medicament for the treatment of psychotic disorders.

The following examples illustrate the invention.

### Example 1:

### (S)-(-)-Decanoic acid 1 -(4-{3-[4-(6-fluoro-benzo[d]isoxazole-3-yl)-piperidine-1-yl]-propoxy}-3-methoxy-phenyl)-ethyl ester

a) 200 ml of a solution of (3aR)-1-methyl-3,3-diphenyl-tetrahydro-pyrrolo[1,2-c][1,3,2]oxazaborole (1M in toluene) is stirred at room temperature under nitrogen. 1.2 equivalent borane-dimethylsulfide complex is added with a syringe. The solution is stirred for 2 further hours at room temperature. The borane complex is then crystallised by addition of 4 vol dry hexane and cooling to -12°C for 1.5 hour. The product is isolated by filtration in a sintered glass funnel and dried in vacuum at 40°C. The boran complex of (3aR)-1-methyl-3,3-diphenyl-tetrahydro-pyrrolo[1,2-c][1,3,2]oxazaborole is obtained (white crystals).
b) 56.36 g of boran complex of (3aR)-1-methyl-3,3-diphenyl-tetrahydro-pyrrolo[1,2-c][1,3,2]oxazaborole (1 equivalent) is dissolved under nitrogen in methylenchloride, and the solution is cooled to 0°C. A 1M solution of 1-(4-{3-[4-(6-fluoro-benzo[d]isoxazol-3-yl)-piperidin-1-yl]-propoxy}-3-methoxy-phenyl)-ethanone (iloperidone; 1 equivalent) in methylenchloride is added via a dropping funnel over 90 minutes while the internal temperature is maintained at 0°C ± 2°C. After the addition is complete, the mixture is stirred at 0°C for 20 hours. The reaction mixture is then poured into precooled methanol (0-5°C) during 1 hour. The solution is warmed to room temperature and stirred until the H₂ evolution ceases. The solution is concentrated by distillation and the residue dried in vacuum, treated with methanol and stirred for about 1 hour at 50°C and an additional hour at 0°C. The product is isolated by filtration and dried under reduced pressure for 3 hours at 50°C. (S)-(-)-1-(4-{3-[4-(6-Fluoro-benzo[d]isoxazol-3-yl)-piperidin-1-yl]-propoxy}-3-methoxy-phenyl)-ethanol is obtained (white crystals).
   [α]_{D} ²⁰ - 19.3° (c=1 in chloroform)
   Mp: 138.2 -138.8°C
c) (S)-(-)-1-(4-{3-[4-(6-Fluoro-benzo[d]isoxazole-3-yl)-piperidine-1-yl]-propoxy}-3-methoxyphenyl)-ethanol (8.57 g, 0.02 mol) is suspended in dichloromethane (150 mL) and pyridine (9.7 mL, 0.02 mol) is added. The reaction mixture is cooled and kept at 0 °C.

Subsequently capric acid chloride (16.4 mL, 0.08 mol) is added slowly. The reaction mixture is further stirred at room temperature for 4 hours. The solution is then poured onto ice water and the liquid fractions are being separated. The aequous fraction is reextracted with methylenchloride. The combined organic fractions are dried and the solvent evaporated. The crude residue is purified by chromatography (neutral aluminum oxide, activity 3) to give (S)-(-)-decanoic acid 1-(4-{3-[4-(6-fluoro-benzo[d]isoxazole-3-yl)-piperidine-1-yl]-propoxy}-3-methoxy-phenyl)-ethyl ester as yellow oil with the optical rotation [α] -42.2 ° (c=0.5, methanol, T= 20 °C, 589 nm); e.e. 97.2 %. The oxalate has a melting point of 99-100.3°C.

The following compounds of formula I are produced analogously to Example 1:

| **Example** | **R** | **13C-NMR C=O (ppm)** | **IR (C=O) cm⁻¹** | **opt. Rot.** | **conc.** | **solv.** |
|---|---|---|---|---|---|---|
| 2 | -(CH₂)₈-CH₃ | 173.150 | 1733 | -42.2 | 0.5 | Methanol |
| 3 | -CH₃ | 169.97 | 1728 | -43.9 | 0.5 | Methanol |
| 4 | -C₃H₇ | 172.47 | 1730 | -40.3 | 0.6 | Methanol |
| 5 | -(CH₂)₆-CH₃ | 173.105 | 1732 | -30.4 | 0.6 | Methanol |
| 6 | -(CH₂)₁₀-CH₃ | 173.161 | 1733 | -4.0.0 | 0.7 | Methanol |
| 7 | -(CH₂)₁₂-CH₃ | 173.138 | 1729 | -37.0 | 0.5 | Methanol |
| 8 | -(CH₂)₁₄-CH₃ | 173.179 | 1729 | -34.5 | 0.6 | Methanol |
| 9 | -CH₂-(CH₂-CH=CH)₆-CH₂-CH₃ | 172.392 | 1734 | | | |
| 10 | -(CH₂)₂-(CH₂-CH=CH)₅-CH₂-CH₃ | 172.008 | 1733 | | | |

## Claims

1. A compound of formula I 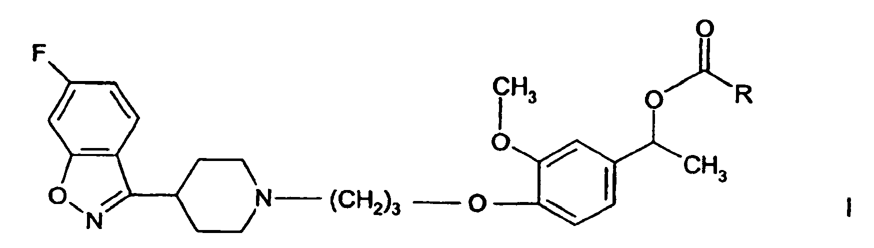 wherein R is (C₁₋₄₀)alkyl or (C₁₋₄₀)alkenyl, in free base or acid addition salt form.

2. A process for the production of the compounds of formula I as defined in claim 1, and their salts, comprising the step of reacting a compound of formula II 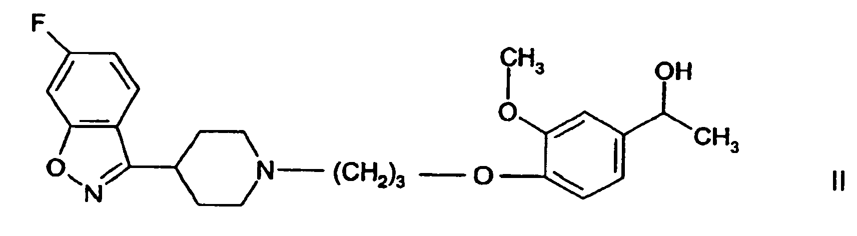 with a compound of formula III 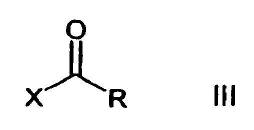 wherein R is as defined in claim 1 and X is halogen, and recovering the resulting compound in free base or acid addition salt form.

3. A compound of claim 1 in free base or pharmaceutically acceptable acid addition salt form, for use as a pharmaceutical.

4. A compound of claim 1 in free base or pharmaceutical acceptable acid addition salt form, for use in the treatment of psychotic disorders.

5. A pharmaceutical composition comprising a compound of claim 1 in free base or pharmaceutical acceptable acid addition salt form, in association with a pharmaceutical carrier or diluent.

6. The use of a compound of claim 1 in free base or pharmaceutically acceptable acid addition salt form, for the manufacture of a medicament for the treatment of psychotic disorders.

## Patentansprüche

1. Verbindung der Formel 1 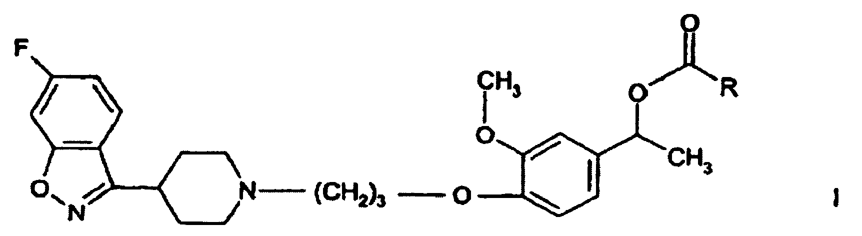 wobei R (C₁₋₄₀)Alkyl oder (C₁₋₄₀)Alkenyl in freier Base- oder Säureadditionssalz-Form ist.

2. Verfahren für die Herstellung der Verbindungen der Formel I, wie in Anspruch 1 definiert, und ihrer Salze mit dem Schritt, eine Verbindung der Formel II 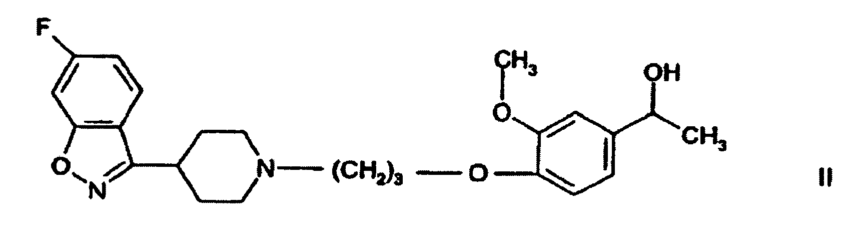 mit einer Verbindung der Formel III 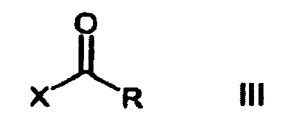 zur Reaktion zu bringen, wobei R wie in Anspruch 1 definiert ist und X Halogen ist, und die resultierende Verbindung in freier Base- oder Säureadditionssalz-Form zurückzugewinnen.

3. Verbindung nach Anspruch 1 in freier Base- oder pharmazeutisch verträglicher Säureadditionssalz-Form zur Verwendung als Pharmazeutikum.

4. Verbindung nach Anspruch 1 in freier Base- oder pharmazeutisch verträglicher Säureadditionssalz-Form zur Verwendung bei der Behandlung von psychotischen Störungen.

5. Pharmazeutische Zusammensetzung mit einer Verbindung nach Anspruch 1 in freier Base- oder pharmazeutisch verträglicher Säureadditionssalz-Form in Zusammenhang mit einem pharmazeutischen Träger oder Verdünnungsmittel.

6. Verwendung einer Verbindung nach Anspruch 1 in freier Base- oder pharmazeutisch verträglicher Säureadditionssalz-Form für die Herstellung eines Medikaments für die Behandlung von psychotischen Störungen.

## Revendications

1. Composé de la formule 1 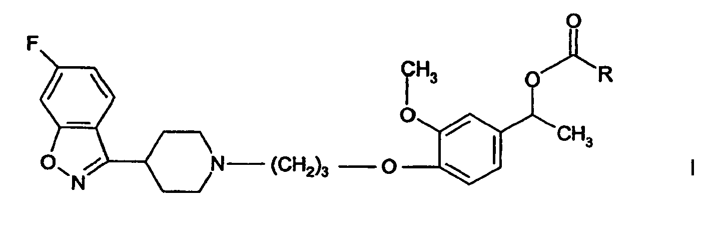 où R est du (C₁₋₄₀)alkyle ou du (C₁₋₄₀)alkényle, en base libre ou sous forme de sel d'addition d'acide.

2. Processus de production de composés de la formule I tel que définis dans la revendication 1, et leurs sels, comprenant les étapes de réaction d'un composé de formule II 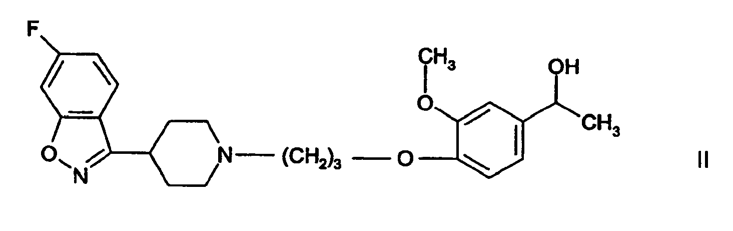 avec un composé de formule III 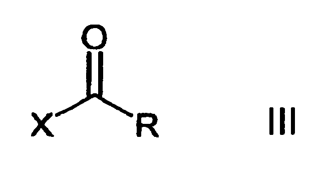 où R est tel que défini dans la revendication 1 et X est halogène et récupération du composé résultant en base libre ou sous forme de sel d'addition d'acide.

3. Composé selon la revendication 1 en base libre ou sous forme de sel d'addition d'acide pharmaceutiquement acceptable pour usage en tant que produit pharmaceutique.

4. Composé selon la revendication 1 en base libre ou sous forme de sel d'addition d'acide pharmaceutiquement acceptable pour usage dans le traitement de désordres psychotiques.

5. Composition pharmaceutique comprenant un composé selon la revendication 1 en base libre ou sous forme de sel d'addition d'acide pharmaceutiquement acceptable en association avec un support ou un solvant pharmaceutique.

6. Utilisation d'un composé selon la revendication 1 en base libre ou sous forme de sel d'addition d'acide pharmaceutiquement acceptable pour la fabrication d'un médicament pour le traitement de désordres psychotiques.
